# EUROPÄISCHE PATENTANMELDUNG

(11) **EP 0 569 768 A1**
(43) Veröffentlichungstag der Anmeldung: **18.11.1993**
(21) Anmeldenummer: 93106762.3
(22) Anmeldetag: 27.04.1993
(51) Int. Cl.: G01N 33/53

(54) **Mittel und Verfahren zur Behandlung von Körperflüssigkeiten bei der Bestimmung von Neopterin**

(30) Priorität: 09.05.1992 DE 4215275
(71) Anmelder: MERCK PATENT GmbH, D-64271 Darmstadt (DE)
(72) Erfinder: Rautenberg, Wilfried, Dr., W-6107 Reinheim (DE); Heubner, Arnulf, Dr., W-6500 Mainz (DE)

(57) **Zusammenfassung**

Die Erfindung betrifft Mittel und Verfahren zur Behandlung von Körperflüssigkeiten bei der immunologischen Bestimmung von Neopterin mit Hilfe eines spezifischen Antikörpers gegen Neopterin und einem Detektionssystem. Das Mittel ist dadurch gekennzeichnet, daß es ein Oxidationsmittel enthält.

## Beschreibung

Die Erfindung betrifft Mittel und Verfahren zur Behandlung von Körperflüssigkeiten bei der immunologischen Bestimmung von Neopterin mit Hilfe eines spezifischen Antikörpers gegen Neopterin und einem Detektionssystem.

Seit der Entdeckung von Neopterin im Harn von Patienten mit malignen oder viralen Erkrankungen im Jahre 1979, hat sich Neopterin als Parameter zur Ermittlung des zellulären Immunstatus bei einer Vielzahl von Patienten mit den verschiedensten Erkrankungen etabliert.

Neopterin gehört zur Gruppe der Pteridine, heterozyklischen Molekülen mit einer weiten Verbreitung im Pflanzen- und Tierreich. Während einige biologische Funktionen von Pteridinen gut dokumentiert sind, ist die biologische Rolle von Neopterin bisher weitgehend unbekannt.

Neopterin wird von Monozyten/Makrophagen nach Induktion durch Interferon-gamma produziert; Interferon-gamma ist wiederum direkt an die Aktivierung der zellvermittelten Immunität gekoppelt und wird von aktivierten T-Lymphozyten gebildet, wenn diese angeregt werden.

Bei einer Reihe von Erkrankungen, wie viralen Infektionen, intrazellulären Parasiten, Septikämie, Transplantatabstoßungen, Autoimmunerkrankungen und Neoplasien spielt die zelluläre Immunität eine entscheidende Rolle. Erhöhte Konzentrationen von Neopterin in Körperflüssigkeiten reflektieren direkt den Aktivierungsgrad des zellulären Immunsystems. Somit ist die Bestimmung der Konzentration von Neopterin in Körperflüssigkeiten sehr gut zur Überwachung des zellulären Immunstatus geeignet.

Die Bestimmung von Neopterin in Körperflüssigkeiten zur Ermittelung des zelluären Immunstatus bei malignen Tumoren und/oder viralen Erkrankungen ist z.B. in EP 12 444 beschrieben. Bisher übliche Verfahren zur Bestimmung von Neopterin in Körperflüssigkeiten, vorzugsweise in Serum und Urin, sind die Hochdruckflüssigkeitschromatographie (HPLC), (J.Chromatogr. 277, 61 (1982)) und der Radioimmunoassay (Chem. Biol. Pteridines, 815 ff. W. de Gruyter, Berlin-New York (1983)). Beide Methoden haben aber einige Nachteile. Neopterin ist mittels HPLC erst nach einem relativ aufwendigen Bearbeitungsverfahren bestimmbar. Jede Analyse dauert dann noch etwa 10 bis 15 Minuten, so daß die Messung einer größeren Probenzahl selbst bei Automatisierung mit einem hohen Arbeits- und Zeitaufwand verbunden ist. Radioimmunoassays dürfen nur in Labors mit Sondergenehmigung durchgeführt werden, und bei der Entsorgung der radioaktiv kontaminierten Arbeitslösungen und Abfälle müssen bestimmte Richtlinien eingehalten werden.

Bei dem der Erfindung Zugrunde liegenden kompetitiven Immunoassay zur Bestimmung von Neopterin sind die Innenoberflächen von Kunststoffgefäßen, Kavitäten einer Mikrotiterplatte, Polystyrolkugeln, Polystyrollatex, Glaskugeln oder magnetische Partikel als Festphase mit einem Neopterin-spezifischen Antikörper beschichtet. Zum Beispiel werden in die Kavitäten einer Mikrotiterplatte die Neopterin-haltigen, zu analysierenden Proben und ein Konjugat aus Neopterin und einem Markerenzym pipettiert. Das Neopterin der Probe und das Konjugat konkurrieren um die limitierten Bindungsplätze auf der Festphase. Hohe Konzentrationen von Neopterin in der Probe führen dabei zu einer geringen Bindung von Konjugat auf der Festphase. Dies führt, nach einem Waschschritt zur Entfernung aller ungebundenen Substanzen, in einer anschließenden für das eingesetzte Markenenzym spezifischen Substratreaktion zu einer geringen Farbstoffentwicklung.

Die Quantifizierung der Neopterinkonzentration in den Proben erfolgt anhand einer Eichkurve, die mit Standards genau definierter Neopterinkonzentrationen erstellt wird. Neopterin ist mit einer mittleren Konzentration von etwa 5 nmol/l im Serum gesunder Personen enthalten; Werte über 10 bis 15 nmol/l gelten als pathologisch. Aufgrund der geforderten hohen Sensitivität des Immunoassays von mindestens 1 nmol Neopterin/l muß die Serumprobe direkt mit den Reaktionspartnern zusammengebracht werden, d.h. das Serum muß im Assay unverdünnt eingesetzt werden. Dabei zeigte sich jedoch, daß die Neopterinbestimmung in frischen Seren, die in der Regel als Probe eingesetzt werden, stets zu hohe Meßwerte lieferte.

Bei der Konzentration von 5 nmol/l z.B. waren die Meßwerte um mehr als 100 % falsch zu hoch.

Die Ausschaltung von Störungen durch Serumbestandteile läßt sich in der Regel durch hohe Verdünnungen des Serums erzielen, was jedoch aufgrund der hier geforderten Sensitivität nicht möglich ist. Auch eine mögliche Denaturierung der Probe durch Hitze oder Zugabe von denaturierenden Agenzien ist für die Neopterinbestimmung ungeeignet. Es stellte sich jedoch heraus, daß der störende Einfluß im Verlauf der Lagerung der Seren zurückging und der Neopteringehalt in älteren Seren richtig gemessen werden kann, wie an der sehr guten Korrelation zu anderen Neopterinbestimmungsverfahren festgestellt werden konnte. Da die Neopterinbestimmung in aller Regel jedoch in frischen Seren durchgeführt werden muß, sind die dabei auftretenden falsch erhöhten Werte für die Diagnostik von größtem Nachteil.

Der Erfindung liegt die Aufgabe zugrunde, ein Mittel und Verfahren zur Behandlung von Körperflüssigkeiten bei der immunologischen Bestimmung von Neopterin zur Verfügung zu stellen, das es erlaubt, Neopterin auch in frischen Körperflüssigkeiten exakt zu bestimmen.

Gegenstand der Erfindung ist ein Mittel zur Behandlung von Körperflüssigkeiten bei der immunologischen Bestimmung von Neopterin mit Hilfe eines spezifischen Antikörpers gegen Neopterin und einem Detektionssystem, das dadurch gekennzeichnet ist, daß es ein Oxidationsmittel enthält.

Ein weiterer Gegenstand der Erfindung ist ein Verfahren zur Behandlung von Körperflüssigkeiten bei der immunologischen Bestimmung von Neopterin mit Hilfe eines spezifischen Antikörpers gegen Neopterin und einem Detektionsystem, das dadurch gekennzeichnet ist, daß die Inkubation aller Reaktionspartner in Gegenwart eines Oxidationsmittels durchgeführt wird.

Überraschenderweise zeigte sich, daß mit dem erfindungsgemäßen Mittel und Verfahren auch in frischen Seren Neopterin mit einem Immunoassay richtig bestimmt werden kann. Das erfindungsgemäße Reagenz beeinträchtigt die immunologische Aktivität von Antikörpern und die enzymatische Aktivität von Markerenzymen nicht und ist bei den für klinische Reagenzien üblichen Lagertemperaturen von 2-8 °C mindestens 1 Jahr lang stabil.

Als Oxidationsmittel eignen sich Kalium- oder Natriumhexacyanoferrat(III), Eisen(III)salz/Chelatisierungsmittel, Peroxidisulfat, Perborat, Nitroprussid, vorzugsweise Kaliumhexacyanoferrat(III) in einer Konzentration von 0,05 bis 100 mmol/l, vorzugsweise 0,5 bis 50 mmol/l. Ähnliche Ergebnisse werden auch mit den anderen Oxidationsmitteln erhalten, z.B. mit der Kombination Eisen(III)salz/Chelatisierungsmittel, vorzugsweise Eisen(III)nitrat/EDTA, in einem Konzentrationsbereich von etwa 0,25 bis 25 mmol/l.

Das erfindungsgemäße Oxidationsmittel wird in einer wässrigen, hochionig gepufferten Lösung mit einem pH-Wert im Bereich von 5 bis 10, vorzugsweise 7 bis 8, eingesetzt. Als Puffersubstanzen können alle in der klinischen Chemie bekannten Puffer oder Pufferkombinationen verwendet werden, die einen pH-Bereich von 5 bis 10, vorzugsweise 7 bis 8, aufrechterhalten können, und keinen störenden Einfluß auf die Testbestandteile haben. Geeignet sind z.B. Phosphatpuffer, TRIS-Puffer, Citratpuffer, HEPES-Puffer, vorzugsweise Phosphatpuffer. Die Pufferkonzentration sollte im Bereich von 50 bis 300 mmol/l, vorzugsweise 150 bis 250 mmol/l, liegen. Das erfindungsgemäße Oxidationsmittel wirkt innerhalb des in der klinischen Chemie üblichen Temperaturintervalls von etwa 15-40 °C optimal.

Die Herstellung des spezifischen Antikörpers gegen Neopterin und die Bindung von Antikörpern an eine feste Phase sind aus der Literatur bekannt. Ebenso ist die Markierung von Antikörpern, Antigenen und Haptenen mit Markerenzymen eine altbekannte Methode. Im erfindungsgemäßen Test werden vorzugsweise Peroxidase und Alkalische Phosphatase als Markerenzyme an das Neopterin gebunden, es können jedoch auch andere Markerenzyme verwendet werden.

Das Detektionssystem enthält die für das betreffende Markerenzym typische Substratlösung aus Substrat, Puffer, Detergenzien, Chromogen usw., wie sie aus der klinischen Diagnostik bekannt sind.

Der Immunoassay zur Bestimmung von Neopterin im Serum wird wie folgt durchgeführt: In die leeren Kavitäten einer Mikrotiterplatte, deren Innenoberfläche mit einem Neopterin-spezifischen Antikörper beschichtet ist, werden die erfindungsgemäße Reagenzlösung, Standards, Kontrollen und Proben pipettiert. Nach einer Inkubationszeit wird die gepufferte Neopterin-Enzymkonjugatlösung hinzugefügt und weiter inkubiert. Nach einem Waschschritt wird eine für das Markerenzym typischen Substratlösung in die Kavitäten pipittiert. Anschließend wird die Substratreaktion mit einem Stoppreagenz gestoppt und die Extinktion des gebildeten Farbstoffs am Absorptionsmaximum gemessen. Die Konzentration der Proben wird aus der erstellten Eichkurve ermittelt. Die Konzentration von Neopterin in der Probe ist umgekehrt proportional zum ermittelten Signal.

### Beispiel 1

### Bestimmung von Neopterin mit einem Konjugat aus Neopterin und Meerrettichperixidase

| Pufferlösung: | Konzentration im Reagenz |
|---|---|
| Phosphatpuffer, pH 7,2 | 250 mmol/l |
| Kaliumhexacyanoferrat (III) | 1,5 mmol/l |
| Natriumchlorid | 150 mmol/l |
| Natriumbenzoat | 10 mmol/l |
| 3-(3-Cholamidopropyl)-dimethylammonio-1-propansulfonat (CHAPS) | 2 mmol/l |
| 5-Chlor-2-methyl-4-isothiazolin-3-on/2-Methyl-4-isothiazolin-3-on | 0,1 % |
| Polyoxyethylensorbitanmonolaurat | 0,05 % |
| Gelatinehydrolysat | 2 % |

### Durchführung der Bestimmung:

In die Kavitäten einer Mikrotiterplatte, deren Innenoberfläche mit einem Neopterin-spezifischen Antikörper beschichtet ist, werden 300 µl einer Waschlösung aus isotoner Natriumchloridlösung und 0,05 % Polyoxyethylensorbitanmonolaurat pipettiert und 30 Minuten stehen gelassen. Nach der vollständigen Entfernung dieser Flüssigkeit werden 50 µl der erfindungsgemäßen Reagenzlösung in jede Kavität pipettiert, gefolgt von je 50 µl Standards, Kontrollen und Proben. Nach einer Inkubationszeit von 30 Minuten bei Raumtemperatur werden 50 µl einer gepufferten Neopterin-Meerrettichperoxidaselösung hinzugefügt und weitere 60 Minuten bei Raumtemperatur inkubiert. Nach einem zweimaligen Waschschritt mit der oben angegebenen Waschlösung werden 150 µl einer Substratlösung von 3,8 mmol/l 2,2'-Azino-di-(3-ethylbenzthiazolin-sulfonat) in 100 mmol/l Citratpuffer, pH 4,3, in die Kavitäten pipettiert. Nach weiteren 30 Minuten bei Raumtemperatur wird die Substratreaktion mit einer 0,095 %igen Natriumazidlösung gestoppt und die Extinktion des gebildeten Farbstoffs bei 405 nm gemessen. Die Konzentration der Proben wird aus der erstellten Eichkurve ermittelt. Die Konzentration von Neopterin in der Probe ist umgekehrt proportional zum ermittelten Signal.

### Beispiel 2

### Vergleich der Meßwerte in frischen und älteren Seren

| Neopterin [nmol/l] | Neopterinmeßwerte [nmol/l] | | | |
|---|---|---|---|---|
| | altes Serum | | frisches Serum | |
| | mit | ohne | mit | ohne |
| | Reagenzlösung nach Beispiel 1 | | | |
| 5 | 5,3 | 5,2 | 4,7 | 11,2 |
| 10 | 11,5 | 10,6 | 10,1 | 17,1 |
| 15 | 16,1 | 16,2 | 16,0 | 21,9 |
| 20 | 21,1 | 21,5 | 20,1 | 26,4 |
| 25 | 24,5 | 25,3 | 24,8 | 29,1 |
| 35 | 35,4 | 36,3 | 37,9 | 41,8 |
| 45 | 46,9 | 45,9 | 43,3 | 50,8 |

Die Tabelle zeigt die Ergebnisse von Versuchen mit steigenden Neopterinkonzentrationen in frischen und älteren Seren. Zu erkennen ist eine wirksame Reduktion der scheinbar erhöhten Neopterinkonzentrationen in frischem Serum durch Einsatz der erfindungsgemäßen Reagenzlösung nach Beispiel 1.

### Beispiel 3

Bestimmung von Neopterin in Abhängigkeit von der zugesetzten Konzentration an Kaliumhexacyanoferrat(III).

Die Durchführung der Bestimmung erfolgt sowohl nach Beispiel 1 (Seren Nr. 1 und 2), als auch analog Beispiel 1, nur daß anstelle von Meerrettichperoxidase Alkalische Phosphatase eingesetzt wird (Seren Nr. 3 und 4).
Substrat: para-Nitrophenylphosphat
Stoppreagenz: 0,1 mol/l Natronlauge
Wellenlänge: 405 nm
Die Ergebnisse der Neopterinbestimmungen in frischen Seren gesunder Probanden in Abhängigkeit von der zugesetzten Kaliumhexacyanoferratkonzentration in der erfindungsgemäßen Reagenzlösung sind in der nachstehenden Tabelle dargestellt.

| Kaliumhexacyanoferrat(III) im Reagenz [mmol/l] | Neopterin-HRP | | Neopterin-AP | |
|---|---|---|---|---|
| | Neopterin[nmol/l] | | | |
| | Serum Nr. | | | |
| | 1 | 2 | 3 | 4 |
| 0 | 12,5 | 16,4 | 18,2 | 19,3 |
| 0,05 | 7,3 | 6,8 | 6,8 | 5,5 |
| 0,10 | 7,2 | 6,6 | 6,6 | 5,2 |
| 0,19 | 7,2 | 6,4 | 6,7 | 4,5 |
| 0,38 | 4,5 | 4,8 | 5,6 | 5,7 |
| 0,75 | 4,1 | 4,9 | 5,9 | 4,1 |
| 1,50 | 4,3 | 5,3 | 5,1 | 4,0 |
| 3,0 | 5,1 | 5,6 | 7,3 | 4,8 |
| 6,0 | 4,1 | 5,5 | 7,1 | 6,2 |
| 12,0 | 4,8 | 6,9 | 8,2 | 6,6 |
| 24,0 | 4,6 | 7,8 | 8,1 | 7,7 |
| 48,0 | 7,1 | 7,5 | 8,7 | 8,8 |

Die Tabelle zeigt, daß frische Seren ohne Zusatz von Kaliumhexacyanoferrat in der Reagenzlösung scheinbar zu hohe Neopterinkonzentrationen - mindestens um Faktor 2 bis 3 - aufweisen. Dabei ist es ohne Bedeutung, ob im Immunoassay ein Neopterin-Peroxidasekonjugat oder ein Neopterin-Alkalische Phosphatase-Konjugat eingesetzt wird. Im erfindungsgemäß bevorzugten Konzentrationsbereich werden Neopterinkonzentrationen gefunden, die eine ausgezeichnete Korrelation zu den etablierten HPLC- und RIA-Bestimmungsmethoden aufweisen. Analoge Ergebnisse erhält man, wenn man anstelle von Kaliumhexacyanoferrat(III) das Natriumhexacyanoferrat(III), Eisen(III)nitrat/EDTA, Kaliumperoxidisulfat, Natriumperborat, Nitroprussid-Natrium, einsetzt.

## Patentansprüche

1. Mittel zur Behandlung von Körperflüssigkeiten bei der immunologischen Bestimmung von Neopterin mit Hilfe eines spezifischen Antikörpers gegen Neopterin und einem Detektionssystem, dadurch gekennzeichnet, daß es ein Oxidationsmittel enthält.

2. Mittel nach Anspruch 1, dadurch gekennzeichnet, daß es als Oxidationsmittel Kaliumhexacyanoferrat(III) enthält.

3. Mittel nach Anspruch 2, dadurch gekennzeichnet, daß es 0.05 bis 100 mmol/l Kaliumhexacyanoferrat(III) enthält.

4. Verfahren zur Behandlung von Körperflüssigkeiten bei der immunologischen Bestimmung von Neopterin mit Hilfe eines spezifischen Antikörpers gegen Neopterin und einem Detektionssystem, dadurch gekennzeichnet, daß die Inkubation aller Reaktionspartner in Gegenwart eines Oxidationsmittels durchgeführt wird.

5. Verfahren nach Anspruch 4, dadurch gekennzeichnet, daß die Inkubation aller Reaktionpartner in Gegenwart von Kaliumhexacyanoferrat(III) durchgeführt wird.
